# EUROPEAN PATENT SPECIFICATION

(11) **EP 2 401 065 B1**
(45) Date of publication and mention of the grant of the patent: **13.12.2017**
(21) Application number: 10707995.6
(22) Date of filing: 26.02.2010
(51) Int. Cl.: B01D 69/12, B01D 71/56, B01D 67/00, C07K 1/34, C07K 1/36

(54) **MEMBRANE WITH SULFONIC GROUPS FOR REMOVING PROTEIN AGGREGATES**
MEMBRAN MIT SULFONSÄUREGRUPPEN ZUM ENTFERNEN VON PROTEINAGGREGATEN
MEMBRANE À GROUPES SULFONIQUES POUR L'ÉLIMINATION D'AGRÉGATS DE PROTÉINES

(30) Priority: 27.02.2009 US 208784 P
(43) Date of publication of application: 04.01.2012
(73) Proprietor: EMD Millipore Corporation, Billerica, MA 01821 (US)
(72) Inventor: KOZLOV, Mikhail, Billerica, MA 01803 (US); RAUTIO, Kevin, Billerica, MA 01803 (US)
(74) Representative: Kirkham, Nicholas Andrew
(86) International application number: PCT/US2010/000578
(87) International publication number: WO 2010/098867

(56) References cited:
- EP-A1- 1 987 892
- WO-A1-2004/103530
- JP-A- 2005 082 728
- US-A1- 2003 146 156
- US-A1- 2003 226 799
- US-A1- 2005 118 479
- US-A1- 2008 216 942
- US-A1- 2009 029 438

## Description

### DESCRIPTION OF THE INVENTION

### Field of the Invention

The present invention relates generally to media for removing biological material from protein solutions. More particularly, to a negatively charged filtration membrane saturated with a polymerized cross-linked coating composition, and methods of making and using the same.

### Background of the Invention

The removal of protein aggregates and viral particles is a frequently encountered separation task during the manufacturing process of protein based therapeutic biological molecules derived from either whole organisms or mammalian cell culture sources. For example, plasma derived protein solutions such as immunoglobulin proteins (IgG) and other proteins (natural or recombinant) such as monoclonal antibodies (mAb), peptides, saccharides, and/or nucleic acid(s) typically contain protein aggregates comprising protein trimers or higher protein polymers that plug and foul viral retention filters and the like.

A common problem in the removal of viral particles from a protein solution by filtration is the use of relatively low capacity ultrafiltration filters which significantly increase the cost, and slows down the filtration operation. Since the size of a viral particle is only about 3-5 times larger than that of a protein molecule, i.e., about 25 nm vs. about 5-10nm, the pore size of the filter has to be carefully chosen to fall in between the sizes of these entities, usually around 20nm. Thus, protein aggregates larger than approximately a trimer or higher protein polymers, as well as denatured proteins, lipids, triglycerides and the like, will not be able to pass through a typical viral retention filter, causing pore blockage and significantly reducing capacity (throughput) of the filter. Even at low concentrations of 0.01 to 0.1 % these unwanted and undesirable constituents will rapidly plug virus retention filters.

However, protein aggregation remains a growing problem in bioprocess manufacturing of protein based therapeutic biological molecules due to increasing solution titers. Protein aggregates form at different stages of the purification process, and in order to maintain adequate throughput of virus retention filters, protein aggregates need to be removed prior to reaching the virus retention filters. Options for selectively removing protein aggregates larger than approximately a trimer or higher protein polymers from a protein solution include utilizing expensive gel chromatography or size exclusion chromatography. The most convenient way to selectively remove protein aggregates is by passing a solution of proteins and/or other biomolecules through a filtration media upstream prior to reaching the viral particle ultrafiltration retention filter located downstream, whereby the protein aggregates are selectively retained upstream, permitting protein monomers in the protein solution to flow through onto the viral retention filter.

One method for removing protein aggregates and viral particles from a protein solution comprises a two-step ultrafiltration process using ultrafiltration membranes in each step. See for example U.S. Patent No. 6,365,395 to Antoniou, wherein protein aggregates are removed from a protein solution upstream, in a first filtration step, and viral particles are removed from the protein aggregate-free protein solution downstream, in the second filtration step.

U.S. Patent No. 7,118,675 to Siwak et al. teaches another two-step filtration process for removing protein aggregates and viral particles from a protein stream wherein the protein stream is first filtered through one or more layers of adsorptive depth filters, charged or surface modified porous membranes, or a small bed of chromatography media to produce a protein aggregate-free protein stream. This is followed by a second step of passing the recovered solution through an ultrafiltration membrane to remove the viral particles.

US 2009/0029438 A1 relates to macroporous gels filling pores of a substrate, that can be used for filtration, adsorption and ion-chromatography, e.g. the use for removal of proteins via ultrafiltration. D1 describes in-situ polymerisation of 2.50 g (≈10wt% of solution) AMPS, 0.372 g (≈15wt% of AMPS) MBA cross-linker, photo-initiator, dissolved in 25 ml of a dioxane:H2O mixture with a volume ratio 9:1 on a support. The solution is applied to the support, which is sandwiched between two sheets and a rubber roller was run over the sandwich to remove excess solution. UV irradiation was used for 1 hour at 350 nm.

One method available for the pretreatment of a protein stream utilizes depth filtration using cellulose media combined with an inorganic filter aid. For example, Viresolve® Prefilter, available commercially from Millipore Corporation, is intended to increase throughput of parvovirus filters when used to pretreat a protein stream prior to virus filtration. However, this prefilter has demonstrated inadequate resistance to caustic treatment, as well as relatively high-organic extractables. Since it is often desirable to clean or sanitize virus filters with a caustic solution, and when a virus filter is combined with a prefilter, the sanitizing process becomes significantly more robust and economical when both can be sanitized in-line by the same treatment. Thus, it is desirable to provide a pretreatment media for a protein solution that can withstand exposure to an aqueous caustic solution (e.g. 0.1 N NaOH for 1 hour) without loss of prefiltration properties. In addition, virus filtration is typically used late in the protein purification process where it is desirable to minimize the amount of organic extractables entering the fluid stream. Depth filters based on cellulose and filter aids have demonstrated significantly higher levels of extractables than membranes, and thus may pose a potential problem for late-stage purification of biological molecules and the like.

Accordingly, it would be desirable to provide filtration media, and devices and processes for using and making the same, for removing protein aggregates and other plugging and fouling constituents from a protein and/or biomolecule containing solution by an upstream prefiltration process that avoids premature plugging of the downstream filtration media such as those utilized in conjunction with the downstream ultrafiltration removal of viral particles from a protein and/or biomolecule containing solution.

### SUMMARY OF THE INVENTION

In response to the above needs associated with the removal of protein aggregates and viral particles in the manufacturing of biomolecule and protein-based therapeutic biological molecules derived from either whole organisms or mammalian cell culture sources, the present invention provides for a negatively charged filtration medium according to claim 1 and a method for making a negatively charged polymerized cross-linked coated porous medium according to claim 7. In certain embodiments, a negatively charged microporous filtration medium having a high charge density that is caustic stable, i.e. resistant to degradation by alkaline solutions, and suitable for use in selectively removing protein aggregates from a protein solution upstream from the ultrafiltration removal of viral particles.

This specification teaches, in certain embodiments, the upstream removal of protein aggregates from a protein stream before downstream viral filtration or other bioprocessing steps of the protein stream. The upstream removal of the protein aggregates reduces the fouling and clogging that would otherwise occur to downstream viral filters, thereby increasing their throughput and flux.

In certain embodiments, this specification teaches a disposable negatively charged microporous medium for removing protein aggregates from a protein solution, thereby eliminating .the cost associated with cleaning and storing the filtering medium between uses, as well as eliminating the cost and time associated with validating such cleaning procedures as required by governmental regulatory agencies.

In certain embodiments, this specification provides a negatively charged coated microporous medium having a high charge density, comprising a microporous substrate and a cross-linked coating composition polymerized in situ on the exterior and interior surfaces of the substrate upon exposure to an electron beam, and in the absence of a chemical polymerization free radical initiator. The cross-linked coating composition is preferably formed from a negatively charged free radical polymerizable acrylamidoalkyl monomer and an acrylamido cross-linking agent.

In certain embodiments, this specification provides a negatively charged coated microporous medium having a high charge density comprising a microporous substrate and a cross-linked coating composition formed from a polymerizable polyfunctional acrylamidoalkyl monomer having negatively charged pendant sulfonic acid containing groups and salts thereof, and a polyfunctional acrylamido cross-linking agent polymerized in situ on the exterior and interior surfaces of the substrate. While polymerization is preferably initiated by exposing the monomer/cross-linker/media system to ionizing radiation such as electron beam in the absence of a chemical polymerization free radical initiator, a suitable chemical polymerization free-radical initiator can also be employed to initiate polymerization.

In an additional embodiment, this specification teaches a negatively charged coated microporous medium comprising a microporous substrate and a cross-linked coating composition formed from a reactant solution comprising a polymerizeable monomer of 2-acrylamido-2-methylpropanesulfonic acid (AMPS) and salts thereof, and a N, N'-methylenebisacrylamide (MBAm) cross-linker polymerized in situ on the inner and outer surfaces of the substrate. The resulting polymerized cross-linked coating composition comprises only amide-amide cross-linking bonds.

In yet an additional embodiment, this specification teaches a negatively charged coated microporous membrane comprising a microporous substrate and a cross-linked polymerized coating composition of AMPS and salts thereof, wherein the cross-linked coating composition is polymerized in situ on the inner and outer surfaces of the substrate

In an additional embodiment, this specification teaches a microporous membrane having a polymerized cross-linked coating composition covering the entire inner and outer surfaces of the microporous substrate, further comprising a supplemental property modifying monomer, which is preferably present in an amount that is less than either of the negatively charged sulfonic acid containing group monomer or the cross-linking agent.

In other embodiments, this specification provides a coated microporous membrane, suitable for use in a prefiltration device, for selectively removing protein aggregates from a protein solution in a dead end normal flow filtration (NFF) process. The protein solution is prefiltered through filtration media including one or more layers of a coated microporous membrane as taught herein, to recover a protein solution substantially free of protein aggregates.

In yet other embodiments, this specification provides methods for the preparation of a negatively charged coated microporous medium having a high charge density, wherein a microporous substrate is coated, contacted, or otherwise saturated with a cross-linked coating composition comprising a negatively charged sulfonic acid group containing a polymerizeable acrylamidoalkyl monomer an acrylamido cross-linking agent, and optionally, a chemical free-radical initiator, deposited onto the entire inner and outer surfaces of the microporous substrate.

In yet other embodiments, this specification provides methods for the preparation of negatively charged coated microporous medium having a high charge density, wherein a microporous substrate is coated with a polymerized cross-linked coating composition comprising polymerizeable monomers of AMPS and salts thereof, and an acrylamido cross-linking agent, such as MBAm over the entire outer and inner surfaces of the microporous substrate, such that the cross-linked coating composition is polymerized in situ on the entire inner and outer surfaces of the microporous substrate.

In another embodiment, this specification provides a method for the preparation of a microporous substrate coated with a polymerized cross-linked coating composition comprising the steps of: a) providing a microporous substrate; b) optionally washing the microporous substrate with a wetting liquid to wet the entire inner and outer surface of the substrate; c) optionally washing the wet surfaces of the microporous substrate with a second wetting liquid to replace the first wetting liquid, leaving the outer and inner surfaces of the substrate wetted with the second liquid; d) contacting the substrate with a reactant solution comprising a negatively charged polymerizable acrylamidoalkyl monomers, an acrylamido cross-linking agent such as MBAm, and optionally a free-radical polymerization initiator; e) depositing the negatively charged cross-linked acrylamidoalkyl monomers coating composition on the inner and outer surfaces of the substrate; f) removing the coated substrate saturated with the cross-linked coating composition from the reactant solution; g) polymerizing the cross-linked acrylamidoalkyl coating composition forming a polymerized cross-linked coating composition on the entire inner and outer surfaces of the substrate; h) rinsing the coated microporous substrate in water and/or organic solvent to remove any unreacted and oligomeric materials; and i) drying the coated microporous substrate.

In yet another embodiment, this specification provides for the selective separation of protein aggregates and viral particles from a protein solution by a two-step filtration process comprising first filtering, under normal flow filtration mode, a protein solution through a prefiltration device having one or more layers of the coated microporous membrane as taught herein, then recovering the protein solution substantially free of protein aggregate. The second filtration step comprises filtering, under either NFF or tangential flow filtration (TFF) mode, the recovered protein solution through one or more ultrafiltration viral removal membranes to retain the viral particles, and permit the passage of the protein solution free of viral particles therethrough.

In other embodiments, this specification provides a negatively charged coated microporous membrane suitable for use in a prefiltration device using a dead end NFF process for selectively removing protein aggregates and other fouling and plugging constituents from an aqueous solution of proteins.

In other embodiments, this specification provides a prefiltration process using a prefiltration device including one or more disposable coated microporous membranes as taught herein, to selectively remove protein aggregates from a biological solution by using NFF process.

In yet another embodiment, this specification provides a process for selectively removing protein aggregates from an aqueous biomolecule containing solution prior to the removal of viral particles, comprising filtering the aqueous biomolecule containing solution through one or more coated microporous membranes as taught herein, under NFF mode of operation, and recovering the biomolecule containing solution substantially free of protein aggregates.

It is another object of this specification to provide a process for selectively removing protein aggregates, denatured proteins, lipids, triglycerides and the like, from an aqueous biomolecule containing solution prior to the downstream removal of viral particles, comprising filtering the aqueous biomolecule solution through one or more prefiltration coated microporous membranes as taught herein, under a NFF mode of operation, and recovering the aqueous biomolecule solution substantially free of protein aggregates, denatured proteins, lipids, triglycerides and the like.

In another embodiment, this specification provides a process for selectively removing protein aggregates from a protein solution using one or more negatively charged coated microporous membranes as taught herein in a NFF mode of operation.

In yet other embodiments, this specification provides a process for selectively removing protein aggregates from a protein a solution using one or more negatively charged coated microporous membranes as taught herein, in a NFF mode, followed by the removal of viral particles by NFF or TFF mode through one or more ultrafiltration viral removal membranes.

This specification further provides a process for treating a biological fluid containing positively charged biomolecules, comprising contacting the biological fluid with a negatively charged coated microporous membrane as taught herein.

This specification further provides a filter device, a chromatography device, and/or a membrane module comprising one or more negatively charged microporous coated substrates having a high charge density as taught herein.

### BRIEF DESCRIPTION OF THE DRAWINGS

FIG. 1 is a flow diagram illustrating a first embodiment of the process of this invention;
FIG. 2 is a flow diagram illustrating another embodiment of the process of this invention;
FIG. 3 graphically depicts the throughput performance measurement of a prefilter/filter combination, according to another embodiment, using a challenge solution of heat-shocked SeraCare IgG (isoelectric point of about 6-9) at 30 psig operating pressure over a range of different conductivity and pH solution conditions;
FIG. 4 depicts a SDS-PAGE analysis of proteinaceous material bound to a membrane prefilter, according to another embodiment, during throughput testing and eluted with 1M NaCl solution;
FIG. 5 depicts a SDS-PAGE analysis of proteinaceous material which was bound to a membrane prefilter according to another embodiment, during throughput testing and eluted with deionized water;
FIG. 6 graphically shows the molecular weight of eluted protein quantified with SEC, wherein no protein was detected in the water elution samples, and a significant amount of high molecular weight (HMW) species were found in the salt elution material, wherein the amount of HMW species is under the level of detection in the feed solution, while 23% of HMW species is eluted from a prefilter membrane according to another embodiment, indicating preferential binding of the aggregates;
FIG. 7 schematically depicts decoupled prefiltration mode of virus removal; and
FIG. 8 schematically depicts spike-across (coupled) prefiltration mode of virus removal.

### DETAILED DESCRIPTION OF THE INVENTION

Before describing the present invention in further detail, a number of terms will be defined.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

As used herein, "AMPS" refers to 2-acrylamido-2-methylpropanesulfonic acid and salts thereof.

As used herein, the terms "biomolecule" or "biological molecule" are intended to mean any organic molecule that is part of a living organism, or analogs thereof. Thus, biomolecules include, but are not limited to, polymers of amino acids, for example peptides and proteins (including antibodies and enzymes), and polymers of nucleotides such as DNA or RNA molecules, and DNA and RNA probes. Also included within the definition of biomolecules are carbohydrates and lipids. It is intended that synthetically produced analogs of each of the foregoing be included in the definition of the term "biomolecule".

As used herein in connection with the membrane substrates and methods of the present invention, the term "clean membrane" means a membrane or membrane substrate that, when produced, has either:
a. less than about 50 micrograms of extractable matter per square centimeter of membrane, and preferably less than about 25 microgram of extractable matter per square centimeter, as determined by the NVR Extraction test described herein; or
b. less than about 25 microgram of extractable matter per square centimeter of membrane as determined by the TOC extractables test described herein.

As used herein, the term "caustic resistant" or "caustic stable" as applied to coated microporous membranes of the invention means a membrane that has no measurable change of permeability or adsorption characteristics after exposure to 0.1 NaOH for two hours at ambient temperature.

As used herein, the term "cross-linked polymer" means a polymer made from two or more monomers that has two or more reactive sites that can take part in a polymerization reaction, or can cross-link separate polymer chains.

As used herein, "MBAm" refers to *N*,*N*'-methylenebisacrylamide.

As used herein "NFF" refers to normal flow filtration.

The term "polymer" as used herein is meant to include polymeric compositions formed from one or more polymerizeable monomers.

As used herein, the term "polyfunctional monomer" means monomers which have more than one unsaturated functional group.

As used herein, the term "reactant solution" means a solution comprising at least a polyfunctional polymerizable monomer having one or more negatively charged sulfonic acid containing groups and salts thereof, and a polyfunctional acrylamido cross-linking agent. An example of a preferred embodiment of the reactant solution is an aqueous solution comprising polymerizable monomers of AMPS and salts thereof, and a MBAm cross-linking agent.

As used herein, the term "surface" as applied to the surface coatings of the membranes and methods of the invention shall mean the entire surface area of a microporous media or membrane, including external or outer surfaces and internal or inner surfaces of the microporous media or membrane. The terms "external surface" or "outer surface" means a surface that is exposed to view, for example either of the planar or microporous surfaces of a membrane. The term "internal surface" or "inner surface" is intended to denote the internal surface of a microporous network, i.e., the interstitial area, of a microporous media or membrane.

As used herein "TFF" refers to tangential flow filtration.

### Microporous Substrates

With respect to the present invention, we will define ultrafiltration substrates as compared to substrates membranes based on the definitions of the International Union of Pure and Applied Chemistry (IUPAC), "Terminology for membranes and membrane processes" published in Pure Appl. Chem., 1996, 68, 1479, as follows:
microfiltration: pressure-driven membrane-based separation process in which particles and dissolved macromolecules larger than 0.1 µm are rejected; and
uitrafiltration: pressure-driven membrane-based separation process in which particles and dissolved macromolecules smaller than 0.1 µm and larger than about 2 nm are rejected.

As used herein, the terms "microfiltration" or "microporous" when used in connection with a membrane, substrate, filter or medium can be in any of several forms, including, but not limited to sheets, tubes, and hollow fibers.

As used herein, the term "ultrafiltration" when used in connection with a membrane, substrate, filter or medium can be in any of several forms, including, but not limited to sheets, tubes, and hollow fibers.

Porous membranes useful in the practice of the present invention are classified as symmetric or asymmetric, referring to the uniformity of the pore sizes across the thickness of the membrane, or, for a hollow fiber, across the microporous wall of the fiber. As used herein, the term "symmetric membrane" means a membrane that has substantially uniform pore size across the membrane cross-section. The term "asymmetric membrane" means a membrane in which the average pore size is not constant across the membrane cross-section.

For example, in asymmetric membranes pore sizes can vary smoothly or discontinuously as a function of location through the membrane cross-section. As will be appreciated, included within the definition of "asymmetric membranes" are membranes that have a ratio of pore sizes on one external surface to those on the opposite external surface that are substantially greater than one.

Representative membranes for use in the present invention taught herein include those formed from of microporous membranes or substrates which are negatively charged, and which may have a surface chemistry (such as hydrophilicity or hydrophobicity), such as taught in U.S. Patent Nos. 5,629,084 to Moya and 4,618,533 to Steuck. A wide variety of microporous substrates made from a wide variety of materials are useful in the practice of the present invention. Examples of such microporous substrates include polysaccharides, nonwoven fabric, ceramic, metal, cotton, cellulose including cellulose/silica blends as well as cellulose derivatives such as cellulose acetate, fiberglass, polymer-based and combinations thereof.

In a preferred embodiment as taught herein the microporous substrate for receiving the cross-linked coating composition is a microporous polymer-based membrane.

Representative polymers that can be used to manufacture the microporous substrate and membranes useful in the present invention include, but are not limited to, substituted or unsubstituted polyacrylamides, polystyrenes, polymethacrylamides, polyimides, polyacrylates, polycarbonates, polymethacrylates, polyvinyl hydrophilic polymers, polystyrenes, polysulfones, polyethersulfones, copolymers or styrene and divinylbenzene, aromatic polysulfones, polytetrafluoroethylenes (PTFE), perfluorinated thermoplastic polymers, polyolefins, aromatic polyamides, aliphatic polyamides, ultrahigh molecular weight polyethylenes, polyvinylidene difluoride (PVDF), polyetheretherketones (PEEK), polyesters, and combinations thereof.

The microporous substrates useful in the present invention may also be made from chromatography media including size exclusion media, ion exchange media, and hydrophobic or hydrophilic media. In a particularly preferred embodiment, the microporous membrane substrate is a polyethersulfone membrane. Those skilled in the art will readily be able to identify other polymers useful in the formation of microporous substrates suitable for the present invention. In embodiments where the microporous membrane substrate is hydrophilic, the preferred materials for the substrate include polyamides, cellulose acetate and cellulose.

The polymerized cross-linked coating composition

The polymerized cross-linked coating composition described herein is formed from a reactant solution comprising at least a polymerizable polyfunctional acrylamidoalkyl monomer having one or more negatively charged pendant sulfonic acid containing groups and/or salts thereof, and a polyfunctional acrylamido cross-linking agent. The reactant solution is an aqueous solution comprising polymerizable monomers of 2-acrylamido-2-methylpropanesulfonic acid (AMPS) and salts thereof, and a polyfunctional acrylamido cross-linking agent such as N, N'-methylenebisacrylamide (MBAm), wherein the resulting polymerized cross-linked coating composition comprises only amide-amide cross-linking bonds.

The reactant solution preferably comprises an aqueous solution of polymerizable monomers of 2-acrylamido-2-methylpropanesulfonic acid (AMPS) and salts thereof, and N, N'-methylenebisacrylamide (MBAm) cross-linkers. The AMPS monomer is present in the reactant solution at a concentration between 1 wt% and 20 wt%, preferably between 3 wt% and 6 wt% based upon the weight of the reactant solution. MBAm cross-linking agent is present in the reactant solution in a concentration between 5 wt% and 100 wt% based upon the weight of the AMPS monomer.

The cross-linked coating composition is polymerized in situ on the surface of the microporous substrate, as well as the inner pore walls, in the absence of any chemical polymerization free radical initiator, upon exposure to electron beam radiation. Preferably, the polymerized cross-linked coating composition covers the entire outer and inner surfaces of the microporous substrate.

The cross-linked coating composition may further comprise a supplemental property modifying monomer, which is preferably present in an amount that is less than either of the polyfunctional acrylamidoalkyl monomer having a negatively charged pendant sulfonic acid containing group, or the polyfunctional acrylamido cross-linking agent.

Process of making the negatively charged coated porous substrate

The reactant solution is applied onto the outer and the inner surfaces the microporous substrate, such that the substrate is saturated with the reactant solution throughout its entire outer and inner surfaces. The desired deposition of the reactant solution onto the inner and outer surfaces of the microporous substrate is effected as a direct coating, and does not require or utilize an intermediate binding chemical component or moiety such as an amino acid or the like.

It is preferred to use a microporous substrate that readily wets with polymerization solution. This minimizes time and expense related to pre-wetting non-wettable substrates with organic solvent and performing solvent exchange.

However, when a non-wettable microporous substrate is used, in order to saturate substantially the entire inner and outer surfaces with the polymerized cross-linked/grafted polymer composition, the first step in forming the coated microporous substrate preferably comprises washing the substrate with a solvent composition such as a mixture of water and an organic solvent that does not swell or dissolve the microporous substrate, and which wets the outer surfaces of the microporous substrate as well as the inner walls of the pores.

Suitable water-organic solvent compositions for this purpose include methanol/water, ethanol/water, acetone/water, tetrahydrofuran/water or the like. The purpose of this wetting step is to assure that the polymerizable AMPS monomer and salts thereof and MBAm cross-linker, subsequently contacted with the microporous substrate, wet the entire inner and outer surfaces of the microporous substrate. This preliminary wetting step can be eliminated when the reagent bath, described below, itself functions to wet the entire surface of the microporous substrate. This can be effected when the reagent bath contains a high concentration of organic reactants, for example 15% by weight or higher.

Subsequent to wetting the microporous substrate, a reagent bath comprising the free radical polymerizable AMPS monomer and salts thereof, and MBAm cross-linker in a solvent, is contacted with the microporous substrate, thereby saturating the inner and outer surfaces of the substrate. The particular solvent employed for the reagent bath will depend upon the particular polymer or material utilized to form the microporous substrate. All that is necessary is that the AMPS monomer and MBAm cross-linker dissolve in the solvent, that the solvent does not attack the microporous substrate, and that the solvent does not negatively affect the polymerization reaction. Representative suitable solvents include water or organic solvents such as alcohols, esters, acetone or compatible aqueous mixtures thereof.

After the microporous substrate is immersed in the reactant solution, such that the inner and outer surfaces are thoroughly saturated with the reactant solution, the microporous substrate is removed from the solution to effect the polymerization reaction outside of the solution so that the polymerizable AMPS monomer is not wasted. Thus, the reaction can be conducted batchwise or continuously. When operating as a continuous process, a sheet of microporous substrate is saturated with the reactant solution and then transferred to a reaction zone where it is exposed to energy from the electron beam to effect the polymerization reaction.

Polymerization can be initiated by employing ionizing radiation in the absence of a chemical polymerization free radical initiator, or not according to the invention a free-radical chemical initiator can be used instead of the ionizing radiation.

When a free-radical initiator is used, it is added to the polymerization solution prior to wetting the microporous substrate, typically in the amount for 0.01 to 1%. Depending on the chemical nature of the free-radical initiator, the polymerization can be initiated by heat or UV irradiation. An example of UV-activated initiator (i.e. photoinitiator) is 1-[4-(2-Hydroxyethoxy)-phenyl]-2-hydroxy-2-methyl-1-propane-1-one (available from Ciba Specialty Chemicals, Basel, Switzerland, under the trade name Irgacure® 2959).

When a chemical free-radical polymerization initiator is not utilized to polymerize the reactant solution, the polymerization, which occurs in situ, relies upon exposing the reactant solution saturating the inner and outer surfaces of the substrate to electron beam radiation, at a dose of at least about 0.1 Mrads to about 6 Mrads, in order to effect polymerization of the AMPS monomers, resulting in a microporous substrate coated on its enter inner and outer surfaces with a polymerized cross-linked coating composition. It has been found that complete coating of the microporous substrate with the cross-linked polymer can be effected without the need of a chemical free radical polymerization initiator.

After the desired dose of radiation has been delivered by the electron beam, the coated microporous substrate is rinsed in water and/or methanol to remove unreacted and oligomeric materials. The coated substrate is then dried and tested for rewet, flow and other properties.

The following examples illustrate the present invention and are not intended to limit the same.

In the following examples, the following applies:
Membrane throughput, which is expressed in volume per unit membrane area, is the volume of solution that can be filtered through the membrane before the flux declines (for constant pressure mode) or pressure increases (for constant flow mode) beyond a practical threshold. In the following examples, the number V₇₅ is the throughput of the combination of prefilter and the virus retentive filter when the initial flux was reduced by 75%, when the experiment is conducted at constant pressure of 2.07 bar (30 psi).

### Flow Time

Flow time is a measure of the effect of the coating thickness on the substrate pore size. If the coating is very thin, then the change in the pore size of the substrate is small and the change in the flow time will be small. If the coating thickness is large, then the change in the substrate pore size and in the flow time will be large. Flow time of the substrate is measured before and after modification to determine the degree of pore restriction that has occurred. The percent of the original flow that is retained by the modified substrate is calculated. Generally, the greater the retention of original flow, the more desirable is the substrate.

The flow time is measured under specific and reproducible conditions. The standard method for determining flow time used herein is to measure the time in seconds required for 500 ml of water to flow through a 47 mm disk of substrate in a glass filter holder at 69.85 cm (27.5 inches) mercury vacuum.

Aggregate-containing solution of proteins was generated according to the procedure described in G.R. Bolton et al., Biotechnol. Appl. Biochem. (2006) 43, 55-63. A solution of 0.1 g/l human IgG (HS-475; SeraCare Life Sciences, Oceanside, CA, U.S.A.) in 50 mM acetate buffer (pH 5.0, 100 mM NaCl) was heated at 60°C for 1 h to denature the protein and produce aggregates. This solution was spiked back into the non-denatured IgG at 9 vol. %. This solution was used throughout the examples to measure membrane throughput.

The ability of a coated substrate to wet with water spontaneously is an important property of a coated substrate. The rewet time is determined by dropping the coated substrate onto water and measuring the time in seconds for the coated substrate to wet through. This is observed visually by the coated substrate becoming darker as it wets.

### General Procedure For Coating the Substrate

The following procedure describes a general method for the treatment of the coated microporous substrate by electron beam radiation to produce a negatively charged coated microporous substrate having a high charge density. The substrate is wetted in methanol, rinsed in water, and soaked in an aqueous polymerizeable AMPS monomers/MBAm cross-linkers reactant solution for several minutes to assure complete exchange. If the aqueous polymerizeable AMPS monomer/MBAm cross-linker reaction solution is capable of wetting the substrate directly, the prewet exchange steps are not necessary.

The electron beam technology used to initiate polymerization of AMPS monomers on the surfaces of the substrate include for example, methods described in U.S. Patent No. 4,944,879 to Steuck. Steuck, for example, teaches a web or individual sample passed through a curtain of electrons generated by an electron beam processor. The processor delivers the desired dose from about 100 kV to about 200 kV. The moving web or sample is transported at a speed suitable to give the desired exposure time under the curtain. Exposure time, combined with dose, determines the dose rate. Typical exposure times are from about 0.5 seconds to about 10 seconds. Dose rates are from 0.01 kGy (kiloGray) to 6 kGy, i.e. 0.1-6 Mrads.

After the desired dose of radiation has been delivered by the electron beam, the treated microporous substrate is rinsed in water and/or methanol to remove unreacted and oligomeric materials. The substrate is then dried and tested for rewet, flow and other properties.

The following procedure describes a general method for the treatment of the coated microporous substrate by using a photoinitiator to produce a negatively charged coated microporous substrate having a high charge density. The substrate is wetted in methanol, rinsed in water, and soaked in an aqueous polymerizeable AMPS monomers/MBAm cross-linkers reactant solution containing a free radical photoinitiator for several minutes to assure complete exchange. If the aqueous polymerizeable AMPS monomer/MBAm cross-linker reaction solution is capable of wetting the substrate directly, the prewet exchange steps are not necessary.

The photoinitiators used to initiate polymerization of AMPS monomers on the surfaces of the substrate include for example, those described in J.-P. Fouassier, Photoinitiation, Photopolymerization, and Photocuring: Carl Hanser Verlag, Munich, Germany, 1995, pp. 20-93, and in Table 3-1, p. 21.

AMPS polymerization is effected by exposing the porous substrate saturated with AMPS/cross-linker/photoinitiator solution to ultra-violet, visible, or infrared radiation, to the dose sufficient to effect decomposition of the photoinitiator and generation of free radicals. A suitable source of ultra-violet radiation may include UV conveyor with two UV light sources, one on top and one on the bottom, as manufactured by Fusion UV Systems, Inc. (Gaithersburg, MD).

After the desired dose of radiation has been delivered by light source, the treated microporous substrate is rinsed in water and/or methanol to remove unreacted and oligomeric materials. The substrate is then dried and tested for rewet, flow and other properties.

### Using the negatively charged coated porous substrate

In one embodiment, this specification provides a process for selectively removing protein aggregates including, but not limited to, protein trimers and higher protein polymers, as well as other plugging and fouling undesired constituents from a protein and/or biomolecule containing solution.

In another embodiment of this specification a protein solution is first filtered with a retentive media to selectively retain protein aggregates comprising protein trimers and higher protein polymers while permitting passage of protein monomers therethrough. This filtration step is effected using a device of one or more layers of an adsorptive membrane. When utilizing these materials, substantially complete protein aggregate removal is effected while permitting recovery of greater than about 85% protein monomer, preferably greater than about 90% protein monomer, most preferably greater than 98% protein monomer.

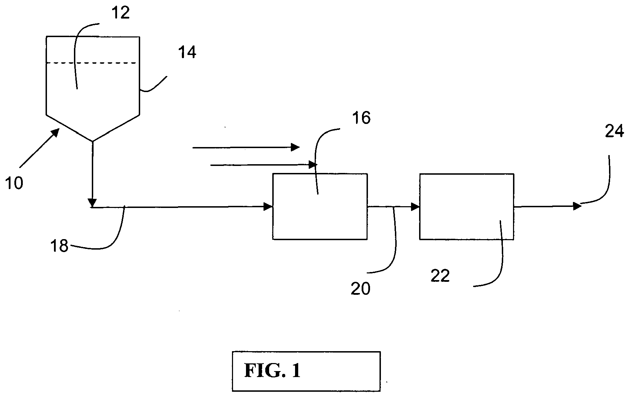

FIG. 1 depicts one of the preferred embodiments of the process of this invention 10 utilizing a constant pressure mode of filtration. A protein solution 12 is retained by pressurized reservoir 14 and is pumped to the filtration media unit 16 by the pressure in the tank through conduit 18. The solution is subjected to a normal flow mode of filtration with the aggregates being retained by the media and the aggregate free solution discharged as the filtrate from the first step 10. The filtrate is passed through conduit 20 for further downstream processing such as the second step of filtration 22 (explained in detail below), and then to an outlet conduit 24. By operating in this manner, protein aggregates are retained by media unit 16 while protein monomers are passed through media 16.

Alternatively, one could use a pump to create the constant pressure of the system, although it is not preferred, as the pump output would need to be carefully controlled to a constant pressure via valves or pump speed and would require a feedback system to ensure that the pressure is kept constant.

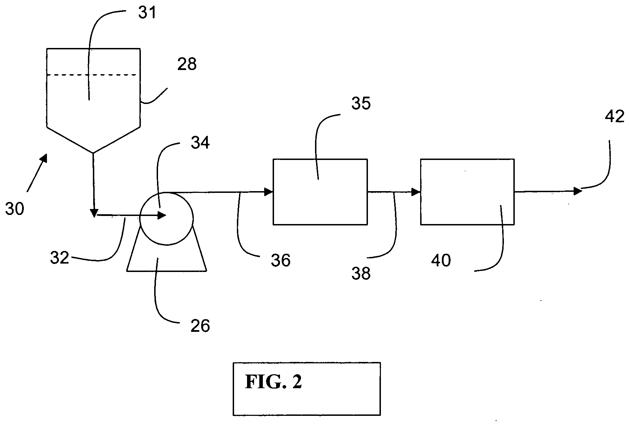

Another embodiment of this specification is shown in FIG. 2 in which a constant flow mode of operation is used. In this system one uses a pump 26 located between the reservoir 28 (typically non-pressurized, as compared to the pressurized reservoir 14 of the embodiment in FIG. 1) and the first filtration step 30 to maintain a constant flow. A protein solution 31 is pumped through conduit 32 to pump inlet 34, and then pumped through conduit 36 to the first filtration step 30. The protein solution 31 is subjected to a normal flow mode of filtration with the aggregates being retained by the filter of the first step 30, wherein the aggregate free solution is discharged as filtrate from the first step 30. The filtrate is passed through conduit 38 for further downstream processing such as the second step of filtration 40 (explained in detail below), and then to an outlet conduit 42.

If desired, one can add a recirculation loop (not shown) at the outlet (not shown) of the first filtration step 30 and recirculate the filtrate through filtration step 30 one or more additional times, to further reduce the aggregate level in the filtrate. Use of a valve (not shown) is the simplest means for controlling the flow between the recirculation loop and the downstream conduit. However, additional recirculation passes are generally unnecessary and increase manufacturing time and costs unnecessarily. It has been found that one recirculation pass is typically sufficient.

In the second filtration step (22 or 40), one conducts a viral removal filtration after the removal of aggregate. Viruses are removed from the aggregate free solution by either a normal flow filter (NFF) or a tangential flow filtration (TFF) filter such as is described in U.S. Pat. No. 6,365,395, filed Nov. 3,2000.

A protein solution is first prefiltered through a negatively charged coated microporous medium prefilter having a high charge density as taught herein, for the removal of protein aggregates. Prefiltration is preferably effected using a dead end NFF mode. Dead-end filtration refers to filtration where the entire fluid stream being filtered goes through the filter with no recycle or retentate flow. Whatever material does not pass through the filter either remains on the upper surface of the filter or remains within the filter.

When filtering a protein solution upstream, in a first step, using the coated microporous medium prefilter for removing protein aggregates as taught herein, viral particle retention filters can be utilized downstream in a second step. In one embodiment, the protein aggregate removal filter can be disposed of after use.

When utilizing a second filtration step, such as is described in U.S. Pat. No. 6,365,395 to Antoniou, to selectively retain viral particles, filtration can be effected with one or more ultrafiltration membranes either by TFF or by dead end NFF, wherein the protein solution produced by this two step filtration process is agglomerate-free and viral-free protein. The one or more ultrafiltration membranes retain viral particles while permitting passage of protein monomers therethrough.

Subsequent the TFF viral filtration step, the ultrafiltration membrane can be optionally flushed with water or an aqueous buffer solution to recover any protein retained by the membrane.

When a protein solution comprising protein trimers and higher protein polymers is first prefiltered with the negatively charged microporous coated membrane having a high charge density as taught herein, protein aggregates are selectively retained while permitting passage of protein monomers therethrough. This prefiltration step is effected using a prefilter having one or more layers of a negatively charged coated microporous membranes, wherein substantially complete protein aggregate removal from the protein solution is effected while permitting recovery of greater than about 85% protein monomers, preferably greater than about 90% protein monomers, and more preferably greater than 98%.

The use of the negatively charged coated microporous membranes used in the prefiltration step to remove plugging constituents from a biomolecule solution provides substantial advantages over presently used conventional protein and viral particle separation processes. Since the filtration device of the first step (removing plugging constituents) is operated in the NFF mode, it may be disposable and there is no cleaning process that would be subject to validation procedures and the like. In addition, normal flow mode of operation is less expensive to purchase and operate, as less capital needs to be expended to set up such a system as compared to a TFF ultrafiltration type system. Furthermore, since the membrane utilized in the second filtration step of removing viral particles does not foul or plug with protein aggregates, its useful life is extended.

Another advantage provided by the invention as taught herein includes the coated microporous membrane used in the first prefiltration step does not have to be an ultrafiltration membrane.

Overall, one can see the improvement in throughput and flux obtained with the NFF aggregate removal step. The Vmax was at least 900% greater than that of the Vmax obtained without the NFF aggregate removal step.

The present invention provides a simple means for the upstream removal of protein aggregates from a protein stream and/or solution before downstream viral filtration or other process steps occur. This prefiltation step reduces the fouling and clogging that would otherwise occur to downstream viral retention filters and the like, increasing throughput dramatically. Additionally, this is done without the need for TFF that is more costly to purchase and to run and which needs to be cleaned between uses. The present invention allows one to dispose of the aggregate filter allowing one to eliminate the cost of cleaning and storing the membrane between uses and the cost and time of validating one's procedures in doing so to regulatory agencies such as the FDA.

When removing viral particles from a protein solution substantially free of protein aggregates, the filtrate from the protein aggregate removal step is directed to a second filtration step. The second filtration step utilizes one or more viral retention filtration (typically ultrafiltration) membranes that can be conducted either in the TFF mode or the NFF mode.

In either mode, the filtration is conducted under conditions to retain the viral particles, generally having a 20 to 100 nanometer (nm) diameter, on the membrane surface while permitting passage of protein monomer and a portion of protein dimer through the membrane.

Representative suitable ultrafiltration viral retention membranes used in the second step viral particle removal step include those formed from regenerated cellulose, polyethersulfones, polyarylsulphones, polysulfones, polyimides, polyamides, polyvinylidenedifluoride (PVDF) or the like, and include VIRESOLVE® NFP, VIRESOLVE® Pro, and RETROPORE® membranes available from Millipore Corporation of Billerica, MA, USA. These can be supplied in either a cartridge NFF form, such as VIRESOLVE® NFP viral filters, or as cassettes for TFF, such as PELLICON® cassettes, also available from Millipore Corporation of Billerica, MA, USA.

Viral filters utilized in the process of this invention are characterized by a log retention value (LRV; the negative logarithm of the sieving coefficient) for viral particles and other, particles that increase monotonically with the diameter of the particle; in the size range of interest for viral particles of 20 to 100 nm diameter. Empirically, the LRV increases continuously with the size of the particle projected area (the square of the particle diameter).

When small sized viral particles are removed from a protein solution, satisfactory LRV of at least about 3 are obtained. However, the molecular weight cutoff is reduced thereby reducing protein recovery. Therefore, a membrane that gives satisfactory LRV and protein recovery is preferable. The membranes utilized in the process of this invention are capable of producing an LRV for viral particles of 3 and can extend to as high as about 8 or greater where the viral particle size is between a 10 and 100 nm diameter.

The protein stream free of protein aggregates can then be filtered through one or more ultrafiltration membranes to retain viral particles at a retention level of at least 3 LRV, and allow the passage therethrough of a protein solution free of viral particles and free protein aggregates.

This specification further provides a device, e.g., a filter device, chromatography device, macromolecular transfer device, flow distributor arrangement, and/or a membrane module comprising one or more negatively charged microporous coated substrates as taught herein. The device can be in any suitable form, for example, the device can include a filter element comprising the negatively charged microporous coated substrate as taught herein in a substantially planar or pleated form. In another embodiment, the filter element can have a hollow generally cylindrical form.

If desired, the device can include the prefilter microporous coated membranes as taught herein in combination with upstream and/or downstream support or drainage layers. The device can include a plurality of membranes, e.g., to provide a multilayered filter element, or stacked to provide a membrane module, such as a membrane module for use in membrane chromatography. Filter cartridges can be constructed by including a housing and endcaps to provide fluid seal as well as at least one inlet and at least one outlet. The devices can be constructed to operate in crossflow or tangential flow (TFF) mode, as well as dead-end mode (NFF). Accordingly, the protein containing solution or stream to be treated can be passed, for example, tangentially to a membrane surface, or passed perpendicular to a membrane surface.

In an another embodiment, the microporous coated membranes as taught herein may be used singularly or in groups, such that the protein containing solution contacts one or more prefilter elements either in parallel or series flow. For example, the coated microporous membranes as taught herein can be used in groups, for example stacked multiple layers (generally from 3 to 8) sealed in the same housing. See, for example, U.S. Patent No. 2,788,901 to Boeddinghaus et al. and U.S. Patent No. 5,085,784 to Ostreicher.

One embodiment shown in FIG. 1, depicts a first process step 10, wherein a constant pressure mode of filtration is utilized. A protein solution 12 is retained by pressurized reservoir 14 and is pumped to the filtration media unit 16 by the pressure in the tank through conduit 18. The protein solution 12 is subjected to a normal flow mode of filtration with the protein aggregates being retained by a negatively charged microporous coated media prefilter as taught herein, contained within the filtration media unit 16, and the protein aggregate free solution is discharged as filtrate from the first step 10. The filtrate is next passed through a conduit 20 for further downstream processing, such as a second filtration process step 22 (as explained infra), and wherein the filtrate exits process step 22 via an outlet conduit 24. By operating in this manner, a protein aggregate free solution results from protein aggregates being retained by media unit 16 having the negatively charged microporous coated media prefilter taught herein, while protein monomers and the like pass through the media unit 16.

Alternatively, a pump can be used to create the constant pressure of the system although it is not preferred as the pump output would need to be carefully controlled to a constant pressure via valves or pump speed and would require a feedback system to ensure that the pressure is kept constant.

Another embodiment is shown in FIG. 2, depicts a first process step 30, wherein a constant flow mode of operation is used. In this system a pump 26 is located between the reservoir 28 (typically a non-pressurized reservoir, as compared to the pressurized reservoir vessel 14 of the embodiment shown FIG. 1), and the filtration media unit 35 to maintain a constant flow. The protein solution 31 is pumped through conduit 32 to the pump inlet 34, and pumped through conduit 36 to the filtration media unit 35. Again, the prefilter used in the first step 30 is the negatively charged microporous coated media as taught herein, and also used in FIG. 1. The protein solution 31 is subjected to a normal flow mode of filtration wherein protein aggregates are retained by the prefilter in the filtration media unit 35, and a protein aggregate free solution is discharged as filtrate and passed through conduit 38 for further downstream processing, such as a second filtration process step 40 (as explained infra), and then to an outlet conduit 42. By operating in this manner, a protein aggregate free solution results from protein aggregates being retained by media unit 35 having the negatively charged microporous coated media prefilter taught herein, while protein monomers and the like pass through the media unit 35.

A recirculation loop (not shown) can be located at an outlet (not shown) of the first filtration step (10, 30) and recirculate the filtrate through the first filtration step one or more additional times to further reduce the protein aggregate level in the filtrate if needed. Use of a valve (not shown) is the simplest means for controlling the flow between the recirculation loop and the downstream conduit. It has been found that one recirculation pass is sufficient. Additional recirculation passes are generally unnecessary and increase manufacturing time and costs unnecessarily.

It is hypothesized that the membrane prefilter of the present invention operates predominantly by ionic binding of positively-charged protein aggregates on its negatively-charged surface. Based on this understanding of how the mechanism of the prefilter is thought to operate, operating conditions, choosing validation strategies, and addressing potential troubleshooting issues may appropriately be determined.

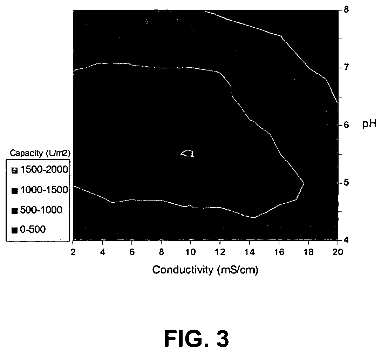

The mechanism of the prefilter operation can be investigated by analyzing the prefilter's performance in a range of solution conditions. For example, conductivity and pH of a challenge solution were varied and the throughput of the prefilter/filter combination was measured. Heat-shocked SeraCare IgG (isoelectric point or "pl" of about 6-9) (SeraCare Life Sciences, Inc., Milford, MA, USA) was used in the challenge solution at 30 psig operating pressure. As depicted in FIG. 3, the prefilter is most effective in a pH range below the pl of aggregates, and a conductivity range of about 2-16 mS/cm. The net positive charge on the aggregates is reduced as the pH approaches the pl, and overall charge interactions are weakened as the conductivity is increased. The relatively poor performance of the prefilter at high pH and high conductivity seems to confirm that ionic interactions are primarily responsible for aggregate removal from the antibody stream.

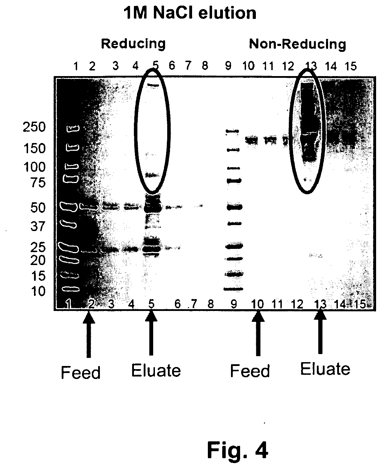

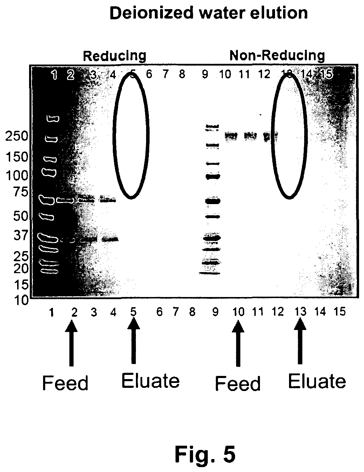

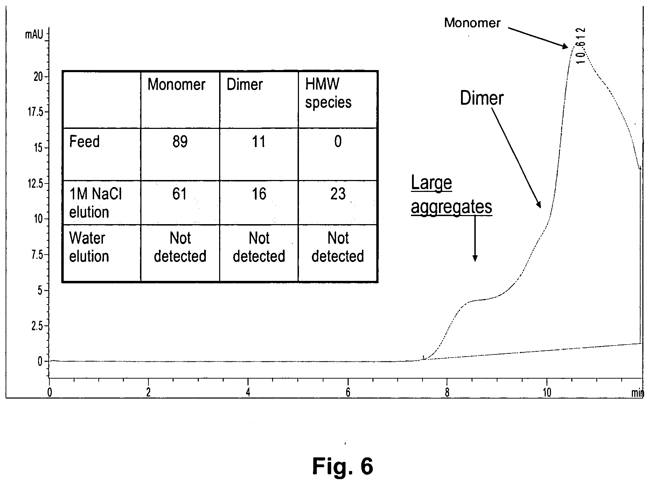

The proteinaceous material bound to the membrane prefilter during the throughput testing was removed (eluted) and analyzed by sodium dodecyl sulfate polyacrylamide gel electrophoresis (SDS-PAGE) and size exclusion chromatography (SEC). Two elution conditions were studied: elution with 1M NaCl solution (shown in FIG. 4) and with deionized water (shown in FIG. 5). From the SDS-PAGE data, no protein elution was observed in deionized water in FIG. 5, while strong antibody and aggregate bands were present in 1M salt eluate FIG. 4. The molecular weight of eluted protein was quantified with SEC as shown in FIG. 6. While no protein was detected in the water elution samples, a significant amount of high molecular weight (HMW) species were found in the salt elution material, shown in FIG. 6. The amount of HMW species is under the level of detection in the feed solution, yet a significant amount (23%) is eluted from the prefilter membrane, indicating preferential binding of the aggregates, also shown in FIG. 6. This further reinforces the assumption that ionic binding of aggregates to the membrane surface is the primary mechanism of prefilter operation.

### EXAMPLES

### Example 1

An aqueous solution was prepared containing 4.5 wt.% of sodium salt of 2-acrylamido-2-methylpropanesulfonic acid (AMPS-Na) and 0.9% of N, N'-methylenebisacrylamide. A hydrophilic polyethersulfone membrane with pore size rating 0.22 um (commercially available as Millipore Express® SHF) was cut into square pieces of 14 cm by 14 cm and submerged in this solution for 30 seconds to ensure complete wetting. The excess of solution was nipped off, and the membrane was exposed to 2 MRads of electron beam radiation under inert atmosphere. The membrane was subsequently rinsed with deionized water and dried in air. Three layers of this membrane were sealed into a vented polypropylene device, with membrane filtration area 3.1 cm². The holder was connected in line to a similar device containing two layers of Viresolve® Pro parvovirus removal membrane. This device combination was first tested for permeability with acetate buffer solution and then for throughput with aggregate-containing solution of polyclonal antibodies prepared as described above. Table 1 shows the combined throughput of this filter combination.

### Example 2

The procedure outlined in Example 1 was followed but AMPS-Na was replaced with 4.0 wt.% of 2-acrylamido-2-methylpropanesulfonic acid (AMPS).

### Example 3

An aqueous solution was prepared containing 4.5 wt.% of sodium salt of 2-acrylamido-2-methylpropanesulfonic acid (AMPS-Na) and 0.9% of N, N'-methylenebisacrylamide. A hydrophobic polyethersulfone membrane with pore size rating 0.22 um (unhydrophilized precursor of Millipore Express® SHF) was cut into square pieces of 14 by 14 cm and submerged into isopropanol for 1 minute, transferred into deionized water for 5 minutes, and then submerged into the monomer solution for 3 minutes. The excess of solution was nipped off, and the membrane was exposed to 2 MRads of electron beam radiation under inert atmosphere. The membrane was subsequently rinsed with deionized water and dried in air. The testing procedure outlined in Example 1 was followed.

### Example 4

The procedure outlined in Example 1 was followed with a hydrophilic polyethersulfone membrane having pore size rating 0.8 um, commercially available under the trade name Micropes from Polypore Inc.'s unit Membrana GMBH, Wuppertal, Germany.

### Example 5, not according to the invention

An aqueous solution was prepared containing 4.5 wt.% of sodium salt of 2-acrylamido-2-methylpropanesulfonic acid (AMPS-Na), 0.9% of N, N'-methylenebisacrylamide, and 0.2% of UV initiator 1-[4-(2-Hydroxyethoxy)-phenyl]-2-hydroxy-2-methyl-1-propane-1-one (available from Ciba Specialty Chemicals, Basel, Switzerland, under the trade name Irgacure® 2959). A hydrophobic polyvinylidenedifluoride membrane with pore size rating 0.65 um was cut into square pieces of 14 cm by 14 cm and submerged into isopropanol for 1 minute, transferred into deionized water for 5 minutes, and then submerged into the monomer solution for 3 minutes. The excess of solution was nipped off, and the membrane was exposed to ultra-violet radiation under inert atmosphere. The membrane was subsequently rinsed with deionized water and dried in air. The testing procedure outlined in Example 1 was followed.

### Example 6 not according to the invention

An aqueous solution was prepared containing 4.5 wt.% of sodium salt of 2-acrylamido-2-methylpropanesulfonic acid (AMPS-Na), 0.9% of N, N'-methylenebisacrylamide, and 0.2% of UV initiator 1-[4-(2-Hydroxyethoxy)-phenyl]-2-hydroxy-2-methyl-1-propane-1-one (available from Ciba Specialty Chemicals, Basel, Switzerland, under the trade name Irgacure® 2959). A hydrophilic ultra-high molecular weight polyethylene membrane with pore size rating 0.65 um was cut into square pieces of 14 by 14 cm and submerged in this solution for 30 seconds to ensure complete wetting. The excess of solution was nipped off, and the membrane was exposed to ultra-violet radiation under inert atmosphere. The membrane was subsequently rinsed with deionized water and dried in air. The testing procedure outlined in Example 1 was followed.

### Example 7

Modified membrane was prepared as described in Example 1. Molded polypropylene devices were manufactured containing 1, 2, 3, and 6 layers of this membrane, and tested as described in Example 1.

### Example 8

Procedure in Example 1 was followed, AMPS-Na concentration was varied from 2 to 6%.

### Example 9

Membrane prepared in Example 1 was exposed twice to gamma irradiation of 30 kGy each, for a total dosage of 60 kGy, i.e. 3 Mrads each, for a total dosage of 6 Mrads. Aggregate removal efficiency was tested before and after exposure as outlined in Example 1.

### Example 10

Membrane prepared in Example 1 was flushed with 0.5N solution of sodium hydroxide for 1 hour at constant pressure 1.72 bar (25 psi), then flushed with deionized water for 60 min at 2.07 bar (30 psi), and tested for aggregate removal efficiency as outlined in Example 1.

### Comparative Example 1

Throughput testing was carried out as described in Example 1, with the exception that no prefiltration step was used. Two layers of Viresolve®Pro parvovirus removal membrane from Millipore Corporation were sealed in a polypropylene vented device. This device was first tested for permeability with acetate buffer solution, and then for throughput with an aggregate-containing solution of polyclonal antibodies prepared as described above. Table 1 shows the combined throughput of this filter combination.

### Comparative Example 2

A Viresolve® Prefilter from Millipore Corporation was used to remove protein aggregates from a protein stream. A commercially available device with 5 cm² filtration area was connected in-line to a Viresolve® Pro-containing device and tested as described above.

**Table 1 (example 5,6 are not according to the invention)**

| Membrane | Permeability, L/(m²h-psi) | V₇₅ throughput of aggregate-containing solution of polyclonal antibodies, L/m² |
|---|---|---|
| | ≈ permeability * 14.50 to obtain L/(m²hbar) | |
| Example 1 | 15.5 | 3549 |
| Example 2 | 14.8 | 2894 |
| Example 3 | 14.1 | 2782 |
| Example 4 | 14.9 | 825 |
| Example 5 | 1.64 | 850 |
| Example 6 | 14.6 | 1602 |
| Example 7 | | |
| 1 Layer | 15.5 | 708 |
| 2 Layers | 14.6 | 1725 |
| 3 Layers | 14.1 | 3549 |
| 6 Layers | 13.7 | 11548 |
| Example 8 | 15.5 | 3879 |
| Example 9 | 15.5 | 3743 |
| Comparative Example 1 | 17 | 75 |
| Comparative Example 2 | 12.8 | 14765 |

One aspect in the development of a virus filtration step for a monoclonal antibody application is determining the strategy for verification that virus removal achieved by this filtration step is done by size exclusion (SEC) only. Virus validation strategies and recommendations are developed by an understanding of virus binding to a prefilter.

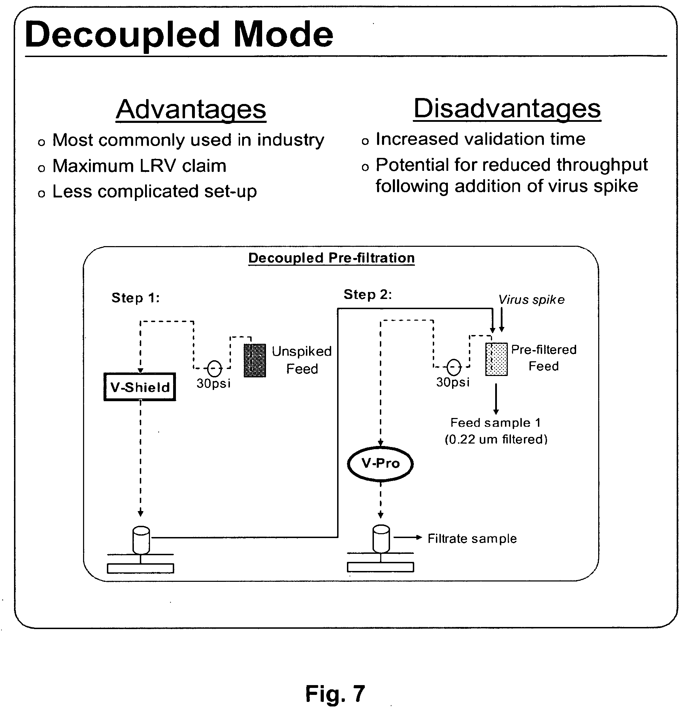

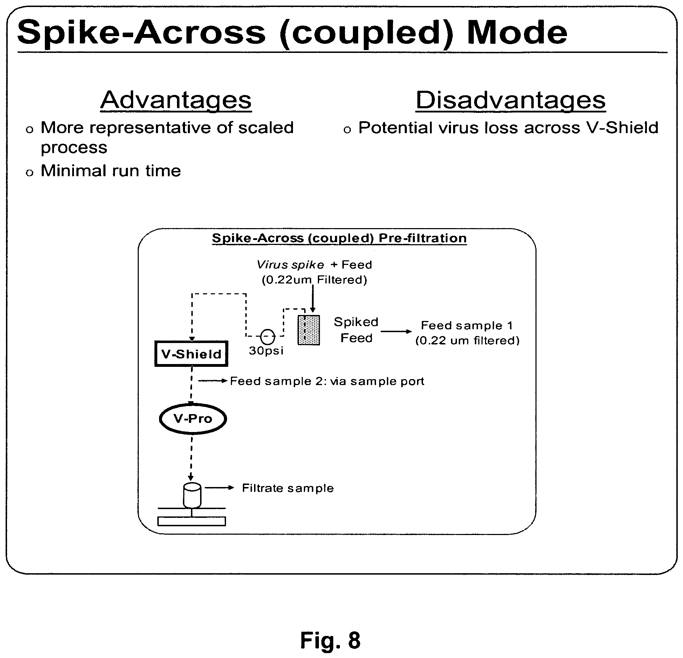

The Viresolve® Prefilter (cellulose fibers with Diatomite) has been shown to remove mammalian viruses by mixed-mode adsorption. Since it is a regulatory requirement to demonstrate virus removal by only size exclusion (SEC) during the validation of the virus filtration step, the Viresolve® Prefilter must be decoupled from the virus filter, as depicted in FIG. 7. The feed material is first filtered through the Viresolve® Prefilter, and then spiked with virus, followed by virus filtration. In many cases, decoupling the Viresolve® Prefilter from the virus filter can lead to reduced capacities even in the absence of virus spikes. FIG. 7 schematically depicts a decoupled prefiltration mode of virus removal. While FIG. 8 schematically depicts a spike-across (coupled) prefiltration mode of virus removal.

A potential benefit of a membrane based, negatively charged prefilter in accordance with this invention in a viral clearance application may be the ability to spike virus through the prefilter during virus validation to maximize capacities and improve ease of use.

If minimal virus removal is observed across the prefiltration step, it may be possible to spike across this prefilter eliminating any potential decoupling effects, as well as providing additional purification of the contaminants from the virus spikes.

An evaluation of the binding of bacteriophage and mammalian virus to this negatively charged prefilter was performed. This was completed in two phases: the first phase was to evaluate bacteriophage as models for the mammalian virus. Since the primary mechanism of removal across the prefilter is charge-based binding, bacteriophages that have similar isoelectric points to the relevant mammalian viruses were chosen.

**TABLE 2: Summary of Tested Viruses**

| **Project Phase** | **Virus** | **Estimated Size(nm)** | **Estimated pl** |
|---|---|---|---|
| Bacteriophage | ΦX-174 | 25 | 6.6 |
| Bacteriophage | PP7 | 25-35 | 4 |
| Mammalian | MMV | 18-26 | 4 |
| Mammalian | XMuLV | 80-110 | 6 |

Feed samples collected from coupled devices resulted in minimal (<0.2logs) bacteriophage (Phi-X 174 and PP7) retention and approximately 0.6-1 logs of MMV across the prefilter. On average, about 2 logs of XMuLV were retained. This is most likely due to the size difference between this virus and the others in the test. These results are suggestive that a virus validation strategy that involves spiking across the prefilter, in the coupled mode, may be possible.

An advantageous property of the negatively charged microporous coated media taught herein includes chemical resistance to withstand contact with highly alkaline solutions in cleaning or sanitation operations without loss of filtration properties, as well as other desirable surface, chemical and mechanical properties

A prefiltration process using the negatively charged coated microporous media as taught herein and operated in the NFF mode to remove plugging and fouling constituents such as protein aggregates from a biological solution, offers several advantages over currently used separation processes, because the coated microporous media is disposable, thereby requiring no cleaning process that would be subject to validation procedures. Additionally, the NFF mode of operation is less expensive to operate compared to a TFF ultrafiltration type systems.

Additionally, the negatively charged coated microporous media taught herein and used upstream from the removal of viral particles from a protein solution inhibits the fouling of the viral particle retention ultrafiltration media utilized downstream by retaining the fouling constituents upstream, thereby extending the useful life of the ultrafiltration viral particle retention media.

The properties of the negatively charged coated microporous media of the present invention make them attractive for use in the prefiltration, detection, separation, and/or purification of biomolecules such as proteins, amino acids, nucleic acids, and viral particles. Examples of nucleic acids include modified or unmodified, synthetic or natural RNA and DNA.

The negatively charged coated microporous media as taught herein also finds use in various applications such as prefiltration and filtration of fluids containing proteins, positively charged atoms, molecules, biomolecules, and particulates, and macromolecular transfer from electrophoresis gels such as the transfer of nucleic acids and proteins from electrophoresis gels to an immobilizing matrix.

The negatively charged coated microporous media as taught herein also find use in the separation or purification of various components present in biological fluids. Thus, for example, the coated microporous media can be used in the purification of human albumin from serum, in the therapeutic fractionation of blood, and the separation of components in genetically engineered cell cultures or fermentation broths. The coated microporous media can also be used in the purification of, for example, viral vaccines and gene therapy vectors such as adeno-associated viral particles.

The present invention provides a simple prefiltration means for the removal of protein aggregates from a protein stream upstream or before viral filtration or other downstream processing steps. The prefiltration means taught herein reduces the fouling and clogging that would otherwise occur to viral retention filters and the like, thereby increasing throughput and flux over traditional filtration processes that do not incorporate the coated microporous membrane as taught herein.

## Claims

1. A negatively charged filtration medium comprising:
a porous substrate coated with a negatively charged polymerized cross-linked acrylamidoalkyl coating, polymerized in situ on the surface of the substrate upon exposure to an electron beam and in the absence of a chemical polymerization free-radical initiator, wherein the coating comprises only amide-amide cross-linking bonds and is formed from a polymerizable acrylamidoalkyl monomer comprising 2-acrylamido-2-methylpropanesulfonic acid and salts thereof, and an acrylamido cross-linking agent comprises N,N'-methylenebisacrylamide, and further wherein the coating is formed from an aqueous reactant solution comprising, based upon the weight of the reactant solution, from 1 to 20 wt% of 2-acrylamido-2-methylpropanesulfonic acid and salts thereof, and based upon the weight of the 2-acrylamido-2-methylpropanesulfonic acid and salts thereof, from 5 to 100 wt% of N, N'-methylenebisacrylamide, **characterised in that** the coating is polymerised by exposing the coating to an electron beam providing a dose of radiation from 0.1 to 6 Mrads for at least 0.1 seconds.

2. The medium according to claim 1, wherein the porous substrate comprises a material selected from the group consisting of substituted or unsubstituted polyacrylamides, polystyrenes, polymethacrylamides, polyimides, polyacrylates, polycarbonates, polymethacrylates, polyvinyl polymers, polysulfones, polyethersulfones, copolymers of styrene and divinylbenzene, aromatic polysulfones, polytetrafluoroethylene (PTFE), perfluorinated thermoplastic polymers, polyolefins, aromatic polyamides, aliphatic polyamides, ultrahigh molecular weight polyethylenes, polyvinylidene difluoride (PVDF), polyetheretherketones (PEEK), polysaccharides, polyesters, cellulose, cellulose derivatives, fiberglass, cotton, ceramics, metals, nonwoven fabrics and combinations thereof.

3. The medium according to claim 1, wherein the porous substrate is washed with a wetting liquid to wet the substrate prior to coating the substrate with the negatively charged polymerized cross-linked acrylamidoalkyl coating.

4. The medium according to claim 1, wherein the porous substrate comprises:
a) a microporous membrane; or
b) one or more pleated microporous membranes.

5. The medium according to claim 1, having a hollow cylindrical form.

6. A negatively charged coated porous membrane comprising the medium according to any one of claims 1 to 5, where the substrate comprises:
a porous membrane having inner and outer surfaces, and the negatively charged polymerized cross-linked acrylamidoalkyl coating having only amide-amide cross-linking bonds is polymerized in situ on the inner and outer surfaces of the porous membrane.

7. A process for making a negatively charged polymerized cross-linked coated porous medium comprising the steps of:
a) providing a porous substrate having inner and outer surfaces;
b) providing an aqueous reactant solution of polymerizable acrylamidoalkyl monomers comprising 2-acrylamido-2-methylpropanesulfonic acid and salts thereof, and the acrylamido cross-linking agent comprise N, N'-methylenebisacrylamide;
c) contacting the porous substrate with the reactant solution;
d) forming a negatively charged cross-linked coating composition having only amide-amide cross-linking bonds, from the reactant solution on the porous substrate wherein the reactant solution comprising, based upon the weight of the reactant solution, from 1 to 20 wt% of 2-acrylamido-2-methylpropanesulfonic acid and salts thereof, and based upon the weight of the 2-acrylamido-2-methylpropanesulfonic acid and salts thereof, from 5 to 100 wt% of N, N'-methylenebisacrylamide;
e) polymerizing the negatively charged cross-linked coating composition in situ on the porous substrate in the absence of a chemical polymerization free radical initiator, **characterised in that** the coating is polymerized by exposing the coating to an electron beam which provides a dose of radiation from 0.1 to 6 Mrads for least about 0.1 seconds; and
f) forming a polymerized negatively charged cross-linked coating on the porous substrate.

8. The process according to claim 7, further comprising step (a1), between steps (a) and (b), of washing the porous substrate with a wetting liquid to wet the substrate.

9. The process according to claim 8, further comprising an additional washing step between steps (a1) and (b), of washing the wet porous substrate with a second wetting liquid to replace the first wetting liquid, wetting the porous substrate with the second liquid.

10. The process according to any one of claims 7 to 9, wherein step (c) comprises immersing the porous substrate in a reactant solution bath, saturating the inner and outer surfaces of the substrate, withdrawing the coated substrate from the reactant solution bath, and removing any excess reactant solution from the coated substrate.

11. The process according to any one of claims 7 to 10, wherein the porous substrate comprises a material selected from the group consisting of substituted or unsubstituted polyacrylamides, polystyrenes, polymethacrylamides, polyimides, polyacrylates, polycarbonates, polymethacrylates, polyvinyl polymers, polysulfones, polyethersulfones, copolymers of styrene and divinylbenzene, aromatic polysulfones, polytetrafluoroethylene (PTFE), perfluorinated thermoplastic polymers, polyolefins, aromatic polyamides, aliphatic polyamides, ultrahigh molecular weight polyethylenes, polyvinylidene difluoride (PVDF), polyetheretherketones (PEEK), polysaccharides, polyesters, cellulose, cellulose derivatives, fiberglass, cotton, ceramics, metals, nonwoven fabrics and combinations thereof.

12. The process according to any one of claims 7 to 11, wherein the porous substrate comprises a microporous membrane.

13. A fluid treatment device comprising:
a housing including at least one inlet, at least one outlet and defining a fluid flow path between the inlet and the outlet, interposed between the inlet and the outlet and across the fluid flow path are a plurality of negatively charged microporous membranes according to claim 4 or claim 6.

14. A process for selectively removing protein aggregates and viral particles from a protein solution containing protein aggregates and viral particles comprising the steps of:
a) filtering a protein solution containing protein aggregates and viral particles through a negatively charged microporous medium according to any one of claims 1 to 5;
b) recovering a protein solution substantially free of protein aggregates;
c) filtering the recovered protein solution through one or more ultrafiltration membranes;
d) retaining viral particles in the one or more ultrafiltration membranes at a level of at least 3 Log Retention Value (LRV); and
e) recovering a protein solution substantially free of viral particles.

15. The process according to claim 14, further comprising the step of flushing protein retained on the one or more ultrafiltration membranes.

16. The process according to claim 15, wherein step (a) filtering comprises normal flow filtration mode.

17. The process according to claim 14, wherein step (c) filtering comprises either normal flow filtration mode or tangential flow filtration mode.

## Patentansprüche

1. Negativ geladenes Filtermedium, umfassend
ein poröses Substrat, das mit einer negative geladenen polymerisierten vernetzten Acrylamidoalkylbeschichtung beschichtet ist, die in situ auf der Oberfläche des Substrats bei Exposition an einen Elektronenstrahl und in Abwesenheit eines radikalliefernden Polymerisierungsinitiators polymerisiert wird, wobei die Beschichtung nur Amid-Amid-Vernetzungen umfasst und aus einem polymerisierbaren Acrylamidoalkylmonomer, das 2- Acrylamido-2-methylpropansulfonsäure und Salze davon umfasst, und einem Acrylamido-Vernetzungsmittel, umfassend N,N'-Methylenbisacrylamid, gebildet ist, und ferner wobei die Beschichtung aus einer wässrigen Eduktlösung gebildet ist, die bezogen auf das Gewicht der Eduktlösung 1 - 20 Gew. % 2-Acrylamido-2-methylpropansulfonsäure und Salze davon und bezogen auf das Gewicht der 2-Acrylamido-2-methylpropansulfonsäure und der Salze davon 5 - 100 Gew. % N, N'-Methylenbisacrylamid umfasst, **dadurch gekennzeichnet, dass** die Beschichtung durch Exposition der Beschichtung an einen Elektronenstrahl, der eine Strahlungsdosis von 0,1 - 6 Mrad über mindestens 0,1 s bereitstellt, polymerisiert wird.

2. Medium nach Anspruch 1, wobei das poröse Substrat ein Material umfasst, das aus der nachfolgenden Gruppe gewählt ist: substituierte oder nicht substituierte Polyacrylamide, Polystyrole, Polymethacrylamide, Polyimide, Polyacrylate, Polycarbonate, Polymethacrylate, Polyvinylpolymere, Polysulfone, Polyethersulfone, Copolymere von Styrol und Divinylbenzol, aromatische Polysulfone, Polytetrafluorethylen (PTFE), perfluorierte thermoplastische Polymere, Polyolefine, aromatische Polyamide, aliphatische Polyamide, ultrahochmolekulare Polyethylene, Polyvinylidendifluorid (PVDF), Polyetheretherketone (PEEK), Polysaccharide, Polyester, Cellulose, Cellulosederivate, Glasfaser, Baumwolle, Keramik, Metalle, Vliesstoffe und Kombinationen davon.

3. Medium nach Anspruch 1, wobei das poröse Substrat mit einer benetzenden Flüssigkeit gewaschen wird, um das Substrat zu benetzen, bevor dieses mit der negativ geladenen polymerisierten vernetzten Acryloamidoalkylbeschichtung beschichtet wird.

4. Medium nach Anspruch 1, wobei das poröse Substrat umfasst:
a) eine mikroporöse Membran oder
b) mindestens eine plissierte mikroporöse Membran.

5. Medium nach Anspruch 1, das eine hohlzylindrische Form aufweist.

6. Negative geladene beschichtete poröse Membran, umfassend das Medium nach einem der Ansprüche 1 - 5, wobei das Substrat umfasst:
eine poröse Membran mit Innen- und Außenflächen, und wobei die negative geladene polymerisierte vernetzte Acryloamidoalkylbeschichtung, die nur Amid-Amid-Vernetzungen aufweist, in situ auf den Innen- und Außenflächen der porösen Membran polymerisiert wird.

7. Verfahren zur Herstellung eines negative geladenen polymerisierten, vernetzten, beschichteten, porösen Medium, umfassend die Schritte:
a) Vorsehen eines porösen Substrats mit Innen- und Außenflächen;
b) Vorsehen einer wässrigen Eduktlösung aus polymerisierbaren Acrylamidoalkylmonomeren, die 2-Acrylamido-2-methylpropansulfonsäure und Salze davon umfasst, wobei das Acrylamido-Vernetzungsmittel N, N'-Methylenbisacrylamid umfasst;
c) Kontaktieren des porösen Substrats mit der Eduktlösung;
d) Bilden einer negativ geladenen, vernetzten Beschichtungszusammensetzung, die nur Amid-Amid-Vernetzungen aufweist, aus der Eduktlösung auf dem porösen Substrat, wobei die Eduktlösung, bezogen auf das Gewicht der Eduktlösung, 1 - 20 Gew. % 2-Acrylamido-2-methylpropansulfonsäure und Salze davon und, bezogen auf das Gewicht der 2-Acrylamido-2-methylpropansulfonsäure und der Salze davon, 5 - 100 Gew. % N,N'-Methylenbisacrylamid umfasst;
e) Polymerisieren der negative geladenen, vernetzten Beschichtungszusammensetzung in situ auf dem porösen Substrat in Abwesenheit eines radikalliefernden chemischen Polymerisierungsinitiators, **dadurch gekennzeichnet, dass** die Beschichtung durch Exposition der Beschichtung an einen Elektronenstrahl polymerisiert wird, der eine Strahlungsdosis von 0,1 - 6 Mrad über mindestens etwa 0,1 s bereitstellt; und
f) Bilden einer polymerisierten, negativ geladenen, vernetzten Beschichtung auf dem porösen Substrat.

8. Verfahren nach Anspruch 7, ferner umfassend einen Schritt (a1) zwischen den Schritten (a) und (b): Waschen des porösen Substrats mit einer benetzenden Flüssigkeit, um dieses zu benetzen.

9. Verfahren nach Anspruch 8, ferner umfassend einen zusätzlichen Waschschritt zwischen den Schritten (a1) und (b): Waschen des nassen porösen Substrats mit einer zweiten benetzenden Flüssigkeit, um die erste benetzende Flüssigkeit zu ersetzen, wobei das poröse Substrat mit der zweiten Flüssigkeit benetzt wird.

10. Verfahren nach einem der Ansprüche 7 - 9, wobei der Schritt (c) umfasst: Eintauchen des porösen Substrats in ein Eduktlösungsbad, Sättigen der Innen- und Außenflächen des Substrats, Entnehmen des beschichteten Substrats aus dem Eduktlösungsbad und ggf. Entfernen der überschüssigen Eduktlösung vom beschichteten Substrat.

11. Verfahren nach einem der Ansprüche 7 - 10, wobei das poröse Substrat ein Material umfasst, das aus der nachfolgenden Gruppe gewählt ist: substituierte oder nicht substituierte Polyacrylamide, Polystyrole, Polymethacrylamide, Polyimide, Polyacrylate, Polycarbonate, Polymethacrylate, Polyvinylpolymere, Polysulfone, Polyethersulfone, Copolymere von Styrol und Divinylbenzol, aromatische Polysulfone, Polytetrafluorethylen (PTFE), perfluorierte thermoplastische Polymere, Polyolefine, aromatische Polyamide, aliphatische Polyamide, ultrahochmolekulare Polyethylene, Polyvinylidendifluorid (PVDF), Polyetheretherketone (PEEK), Polysaccharide, Polyester, Cellulose, Cellulosederivate, Glasfaser, Baumwolle, Keramik, Metalle, Vliesstoffe und Kombinationen davon.

12. Verfahren nach einem der Ansprüche 7 - 11, wobei das poröse Substrat eine mikroporöse Membran umfasst.

13. Vorrichtung zur Flüssigkeitsaufbereitung, umfassend:
ein Gehäuse, das mindestens eine Eintrittsöffnung, mindestens eine Austrittsöffnung umfasst und einen Strömungsweg zwischen Eintritts- und Austrittsöffnung definiert, wobei zwischen Eintritts- und Austrittsöffnung und quer über den Strömungsweg eine Mehrzahl negativ geladener mikroporöser Membranen nach Anspruch 4 oder 6 angeordnet ist.

14. Verfahren zur selektiven Entfernung von Proteinaggregaten und Viruspartikeln au seiner Proteinlösung, die Proteinaggregate und Viruspartikel enthält, umfassend die Schritte:
a) Filtern einer Proteinlösung, die Proteinaggregate und Viruspartikel enthält, durch ein negativ geladenes, mikroporöses Medium nach einem der Ansprüche 1 - 5;
b) Zurückgewinnen einer Proteinlösung, die im Wesentlichen frei von Proteinaggregaten ist;
c) Filtern der zurückgewonnenen Proteinlösung durch mindestens eine Ultrafiltrationsmembran;
d) Zurückbehalten von Viruspartikeln in der mindestens einen Ultrafiltrationsmembran auf einem Niveau von mindestens 3 LRV (Log-Rückhaltewert) und
e) Zurückgewinnen einer Proteinlösung, die im Wesentlichen frei von Viruspartikeln ist.

15. Verfahren nach Anspruch 14, ferner umfassend den Schritt: Spülen des auf der mindestens einen Ultrafiltrationsmembran zurückbehaltenen Proteins.

16. Verfahren nach Anspruch 15, wobei der Schritt (a) Filtern einen Filtermodus mit normalem Durchfluss umfasst.

17. Verfahren nach Anspruch 14, wobei der Schritt (c) Filtern einen Filtermodus entweder mit normaler Strömung oder mit Tangentialströmung umfasst.

## Revendications

1. Milieu de filtration chargé négativement comprenant :
un substrat poreux revêtu d'un revêtement acrylamidoalkyle réticulé polymérisé chargé négativement, polymérisé in situ sur la surface du substrat par exposition à un faisceau électronique et en l'absence d'un initiateur de polymérisation radicalaire chimique, dans lequel le revêtement ne comprend que des liaisons de réticulation amide-amide et est formé à partir d'un monomère d'acrylamidoalkyle polymérisable comprenant de l'acide 2-acrylamido-2-méthylpropanesulfonique et des sels de celui-ci, et un agent de réticulation acrylamido comprend du N,N'-méthylènebisacrylamide, et en outre dans lequel le revêtement est formé à partir d'une solution aqueuse de réactif comprenant, sur la base du poids de la solution de réactif, de 1 à 20 % en poids d'acide 2-acrylamido-2-méthylpropanesulfonique et de sels de celui-ci, et sur la base du poids de l'acide 2-acrylamido-2-méthylpropanesulfonique et des sels de celui-ci, de 5 à 100 % en poids de N,N'-méthylènebisacrylamide, **caractérisé en ce que** le revêtement est polymérisé par exposition du revêtement à un faisceau électronique en fournissant une dose de rayonnement de 0,1 à 6 Mrad pendant au moins 0,1 seconde.

2. Milieu selon la revendication 1, dans lequel le substrat poreux comprend un matériau choisi dans le groupe constitué des polyacrylamides substitués ou non substitués, des polystyrènes, des polyméthacrylamides, des polyimides, des polyacrylates, des polycarbonates, des polyméthacrylates, des polymères de polyvinyle, des polysulfones, des polyéthersulfones, des copolymères de styrène et de divinylbenzène, des polysulfones aromatiques, du polytétrafluoroéthylène (PTFE), des polymères thermoplastiques perfluorés, des polyoléfines, des polyamides aromatiques, des polyamides aliphatiques, des polyéthylènes de poids moléculaire ultra-élevé, du difluorure de polyvinylidène (PVDF), des polyétheréthercétones (PEEK), des polysaccharides, des polyesters, de la cellulose, des dérivés de cellulose, de la fibre de verre, du coton, des céramiques, des métaux, des nontissés et des combinaisons de ceux-ci.

3. Milieu selon la revendication 1, dans lequel le substrat poreux est lavé à l'aide d'un liquide mouillant pour mouiller le substrat avant le revêtement du substrat avec le revêtement acrylamidoalkyle réticulé polymérisé chargé négativement.

4. Milieu selon la revendication 1, dans lequel le substrat poreux comprend :
a) une membrane microporeuse ; ou
b) une ou plusieurs membranes microporeuses plissées.

5. Milieu selon la revendication 1, ayant une forme cylindrique creuse.

6. Membrane poreuse revêtue chargée négativement comprenant le milieu selon l'une quelconque des revendications 1 à 5, où le substrat comprend :
une membrane poreuse ayant des surfaces interne et externe, et le revêtement acrylamidoalkyle réticulé polymérisé chargé négativement n'ayant que des liaisons de réticulation amide-amide est polymérisé in situ sur les surfaces interne et externe de la membrane poreuse.

7. Procédé de fabrication d'un milieu poreux revêtu réticulé polymérisé chargé négativement comprenant les étapes de :
a) fourniture d'un substrat poreux ayant des surfaces interne et externe ;
b) fourniture d'une solution aqueuse de réactif de monomères d'acrylamidoalkyles polymérisables comprenant de l'acide 2-acrylamido-2-méthylpropanesulfonique et des sels de celui-ci, et l'agent de réticulation acrylamido comprend du N,N'-méthylènebisacrylamide ;
c) mise en contact du substrat poreux avec la solution de réactif ;
d) formation d'une composition de revêtement réticulée chargée négativement n'ayant que des liaisons de réticulation amide-amide, à partir de la solution de réactif sur le substrat poreux dans lequel la solution de réactif comprend, sur la base du poids de la solution de réactif, de 1 à 20 % en poids d'acide 2-acrylamido-2-méthylpropanesulfonique et des sels de celui-ci, et sur la base du poids de l'acide 2-acrylamido-2-méthylpropanesulfonique et des sels de celui-ci, de 5 à 100 % en poids de N,N'-méthylènebisacrylamide ;
e) polymérisation de la composition de revêtement réticulée chargée négativement in situ sur le substrat poreux en l'absence d'initiateur de polymérisation radicalaire chimique, **caractérisée en ce que** le revêtement est polymérisé par exposition du revêtement à un faisceau électronique qui fournit une dose de rayonnement de 0,1 à 6 Mrad pendant au moins environ 0,1 seconde ; et
f) formation d'un revêtement réticulé chargé négativement polymérisé sur le substrat poreux.

8. Procédé selon la revendication 7, comprenant en outre l'étape (a1), entre les étapes (a) et (b), de lavage du substrat poreux avec un liquide mouillant pour mouiller le substrat.

9. Procédé selon la revendication 8, comprenant en outre une étape additionnelle de lavage entre les étapes (a1) et (b), consistant à laver le substrat poreux humide avec un second liquide mouillant pour remplacer le premier liquide mouillant, en mouillant le substrat poreux avec le second liquide.

10. Procédé selon l'une quelconque des revendications 7 à 9, dans lequel l'étape (c) comprend l'immersion du substrat poreux dans un bain de solution de réactif, la saturation des surfaces interne et externe du substrat, le retrait du substrat revêtu du bain de solution de réactif, et l'élimination de tout excès de solution de réactif du substrat revêtu.

11. Procédé selon l'une quelconque des revendications 7 à 10, dans lequel le substrat poreux comprend un matériau choisi dans le groupe constitué des polyacrylamides substitués ou non substitués, des polystyrènes, des polyméthacrylamides, des polyimides, des polyacrylates, des polycarbonates, des polyméthacrylates, des polymères de polyvinyle, des polysulfones, des polyéthersulfones, des copolymères de styrène et de divinylbenzène, des polysulfones aromatiques, du polytétrafluoroéthylène (PTFE), des polymères thermoplastiques perfluorés, des polyoléfines, des polyamides aromatiques, des polyamides aliphatiques, des polyéthylènes de poids moléculaire ultra-élevé, du difluorure de polyvinylidène (PVDF), des polyétheréthercétones (PEEK), des polysaccharides, des polyesters, de la cellulose, des dérivés de cellulose, de la fibre de verre, du coton, des céramiques, des métaux, des nontissés et des combinaisons de ceux-ci.

12. Procédé selon l'une quelconque des revendications 7 à 11, dans lequel le substrat poreux comprend une membrane microporeuse.

13. Dispositif de traitement de fluide comprenant :
un logement incluant au moins une entrée, au moins une sortie et définissant un trajet d'écoulement de fluide entre l'entrée et la sortie, interposé entre l'entrée et la sortie et le long du trajet d'écoulement de fluide se trouve une pluralité de membranes microporeuses chargées négativement selon la revendication 4 ou la revendication 6.

14. Procédé d'élimination sélective d'agrégats de protéines et de particules virales d'une solution de protéines contenant des agrégats de protéines et des particules virales comprenant les étapes de :
a) filtration d'une solution de protéines contenant des agrégats de protéines et des particules virales à travers un milieu microporeux chargé négativement selon l'une quelconque des revendications 1 à 5 ;
b) récupération d'une solution de protéines sensiblement exempte d'agrégats de protéines ;
c) filtration de la solution de protéines récupérée à travers une ou plusieurs membranes d'ultrafiltration ;
d) rétention des particules virales dans les une ou plusieurs membranes d'ultrafiltration à un niveau d'au moins 3 LRV (Log Retention Value, logarithme de la valeur de rétention) ; et
e) récupération d'une solution de protéines sensiblement exempte de particules virales.

15. Procédé selon la revendication 14, comprenant en outre l'étape de rinçage de la protéine retenue sur les une ou plusieurs membranes d'ultrafiltration.

16. Procédé selon la revendication 15, dans lequel l'étape (a) de filtration comprend un mode de filtration à flux normal.

17. Procédé selon la revendication 14, dans lequel l'étape (c) de filtration comprend soit un mode de filtration à flux normal soit un mode de filtration à flux tangentiel.
